# EUROPEAN PATENT APPLICATION

(11) **EP 3 667 674 A1**
(43) Date of publication of application: **17.06.2020**
(21) Application number: 18211962.8
(22) Date of filing: 12.12.2018
(51) Int. Cl.: G16H 30/00, G16H 50/20

(54) **METHOD AND SYSTEM FOR EVALUATING IMAGES OF DIFFERENT PATIENTS, COMPUTER PROGRAM AND ELECTRONICALLY READABLE STORAGE MEDIUM**

(71) Applicant: Siemens Healthcare GmbH, 91052 Erlangen (DE)
(72) Inventor: Ionasec, Razvan, 90402 Nuernberg (DE)

(57) **Abstract**

Method for evaluating images of different patients acquired with multiple medical imaging devices (3), comprising the steps of
- after acquisition of each image, determining at least one additional information from the image using data extraction algorithms, the data extraction algorithms comprising at least one artificial intelligence algorithm, and storing a structured dataset comprising the image and the additional information in an image database (6),
- executing application algorithms using artificial intelligence and evaluating at least the additional information of multiple datasets stored in the image database (6) to determine an evaluation information, and
- outputting the evaluation information to a user.

## Description

The invention concerns a method and an analystics system for evaluating images of different patients acquired with multiple medical image devices. Furthermore, the invention relates to a computer program and an electronically readable storage medium.

Medical imaging is an important tool in healthcare. Many different modalities and the respective medical imaging devices (scanners) are well-established, for example magnetic resonance imaging, computed tomography, ultrasound imaging and the like. Once a patient has been imaged, the corresponding images may be viewed and read, wherefore different applications and/or systems have been proposed. Another well-explored field regarding medical images is their storage in storage systems, for example picture archiving and communication systems (PACS) and/or vendor neutral archives (VNA). Regarding findings related to images, these are, for example, communicated in medical reports, stored in electronic health records regarding a particular patient, and the like. In this manner, helpful results for analytics on a larger scale, which may have been derived during image reading or even computer aided diagnosis, may be lost.

Population health applications are an approach to healthcare that aims to improve the health of an entire human population. Population health applications and other related healthcare analytics approaches use health information of a plurality of patients to derive evaluation information, for example statistical information to perform predictions regarding a current case, to search similar cases and to facilitate and/or support clinical studies. These data-driven technologies, in particular using artificial intelligence and/or machine learning, for healthcare analytics applications today try to exploit data extracted from radiology readings, reporting workflows and the like. However, most data available from such sources is provided in an unstructured form and lacks information relevant for the corresponding healthcare analytics application. While medical images may sometimes be stored in, for example, electronic health records, it would be, if at all possible, very complex to derive required higher-order information from such medical images themselves.

Thus, today, no image-centric population health applications or other health care analytics applications usable in an efficient clinical setting are known. The exploitation of the information encapsulated within medical imaging databases is mostly realized by manually retrieving and reviewing known similar cases or in a pre-clinical/research setting using small-scale experiments to investigate various image-centric features within a clinical context.

It is an object of the current invention to provide an efficient approach to healthcare analytics applications, in particular population health applications, yielding high quality results.

This object is achieved by providing a method, an analytics system, a computer program and an electronically readable storage medium according to the independent claims. Advantageous embodiments are described in the dependent claims.

A method for evaluating images of different patients acquired with multiple medical imaging devices according to the invention comprises the steps of
- after acquisition of each image, determining at least one additional information from the image using data extraction algorithms, the data extraction algorithms comprising at least one artificial intelligence algorithm, and storing a structured dataset comprising the image and the additional information in an image database,
- executing application algorithms using artificial intelligence and evaluating at least the additional information of multiple datasets stored in the image database to determine an evaluation information, and
- outputting the evaluation information to a user.

Accordingly, an analytics systems for evaluating images of different patients acquired with multiple medical imaging devices according to the invention comprises
- an information extraction subsystem for receiving the images from the respective medical imaging devices, for determining at least one additional information from the image using data extraction algorithms, the data extraction algorithms comprising at least one artificial intelligence algorithm, and for forming a structured dataset comprising the image and the additional information,
- an image database for storing the structured datasets,
- an application subsystem for executing application algorithms using artificial intelligence and for evaluating at least the additional information of multiple datasets stored in the image database to determine an evaluation information, and
- an output unit for outputting the evaluation information to a user.

According to the invention, an image-centric system is proposed that employs artificial intelligence-based algorithms, that is, at least a part of the data extraction algorithms, to enrich medical imaging databases with higher order structured data, and the same employs artificial intelligence-based algorithms, that is, at least part of the application algorithms, to enable health care analytics applications, in particular population health applications, like, for example, hypothesis testing, population registries and the like, in this manner improving health care management of individual patients and groups of patients. The invention is based on the idea that a lot of potentially useful higher order additional information can be derived from medical images, in particular using artificial intelligence, such that it is proposed to use an information extraction subsystem having a plurality of data extraction algorithms, at least partly artificial intelligence-based, such that suitable data extraction algorithms for a certain image may be selected and applied to evaluate the medical image and derive corresponding additional information from it. Furthermore, the information extraction subsystem structures the derived additional information, that is, higher-order data, to form a structured dataset, in which both the medical image and these higher-order additional information are included. The result is a medical image database containing not only the medical images, but additionally higher-order information which may be readily accessed by healthcare analytics applications. It is important to note that the structured datasets are still image datasets, that is, each structured dataset has the at least one medical image of one examination of a patient at maximum, not all images of a certain patient, which may be spread into different structured datasets, each containing the one or, for example regarding a time series, more medical images of one certain examination. In most cases, each structured dataset will contain one medical image to which the additional information relates, for example a two- or three-dimensional magnetic resonance image or x-ray image, in particular computed tomography image.

Considering the fact that it is estimated that around 90 % of all medical data are medical images, the current analytics systems will enable extraction and organization of higher-order imaging information from large image databases to facilitate image centric healthcare analytics solutions, making available an information resource not yet exploited in population health management today.

The analytics system receives medical images from at least one medical imaging device, preferably multiple medical imaging devices, for example all medical imaging devices of a medical institution or multiple medical institutions. The medical imaging devices may comprise medical imaging scanners of any type and modality, for example computed tomography devices, magnetic resonance devices, ultrasound devices, x-ray devices, nuclear imaging devices and/or any other scanner capable of producing medical image measurements. These medical imaging devices are sending the medical images, that is, any reconstructed and/or pre-processed image and/or raw measurements, to the information extraction subsystem after each examination of a patient.

Preferably, the medical images are provided in a DICOM format, thus usually containing helpful metadata, for example describing the image acquisition and/or certain characteristics of a patient. Generally said, advantageous embodiments of the current invention may provide that at least one patient information describing at least one characteristic of the patient, in particular comprising the age and/or the gender, is additionally stored in the structured dataset, and/or that the image is provided and stored in a DICOM format having metadata, the metadata being used by at least part of the data extraction algorithms.

As for the data extraction algorithms, multiple variants and concrete embodiments have already been proposed in the state of the art which can also be applied to the current invention, in particular regarding the extraction of additional information using artificial intelligence. Generally, the information extraction subsystem consists of a comprehensive and extendable stack (library) of data extraction algorithms, comprising in particular artificial intelligence algorithms using machine learning and related data-driven methods. These data extraction algorithms run on a suitable processing infrastructure to enrich the image data coming from the medical imaging devices with higher order additional information such that the medical images and their associated additional information can be stored as a structured dataset into the image database. The information extraction subsystem thus comprises at least one processor and at least one storage means.

In concrete embodiments, the additional information may comprise geometrical information regarding at least one anatomical structure depicted in the image and/or functional information regarding at least one anatomical structure depicted in the image, in particular derived by a physiological measurement algorithm, and/or at least one abnormality information regarding at least one anatomical structure depicted in the image, in particular comprising at least one diagnostic hypothesis and/or likelihood information.

That is, to extract the additional information, geometrical structures in the image may be analysed. The extracted data may then, for example, include information on landmarks, two- and three-dimensional contours, and/or more complex and composite geometric structures as well as temporal/dynamic variations of these structures. Furthermore, at least one data extraction algorithm may perform physiological measurements, such that the extracted data may comprise measurement such as anatomical/morphological size of organs of sub-structures, functional measurements such as the ejection fraction of the left ventricle or brain activity, hemodynamics measurements such as the blood velocity, the fractional flow reserve or blood perfusion-derived measurements, metabolic activities, and/or any other values that quantify certain physiological aspects. As basic techniques, for example, segmentation approaches and the like may be employed.

Additional information may further be derived from the medical images that relates to abnormalities, in particular abnormalities relating to the current state of the patient, that is, findings, and/or remainders of previous conditions, for example scars and the like. Additional information may, for example, comprise extracted data regarding organ lesions in the lungs, liver or other organs, enlarged or deformed organs or sub-structures, bleedings, airleaks/accumulations, fractures, foreign bodies, abnormal motions and the like. Findings-related additional information may further comprise descriptions of location, size, aspect, density, appearance, thickness, texture, classification, fibrosis, occupation, shape and/or other properties of lesions and/or other findings.

Data extraction algorithms using artificial intelligence may be trained using known machine learning methods, wherein training data may be taken from sufficiently large training databases of medical images and ground truths, that is, annotated extracted additional information. Typically, in an exemplary embodiment, each extracted additional information may be produced by a dedicated data extraction algorithm, which, in case of an artificial intelligence algorithm, has been trained and validated for the corresponding purpose. In preferred embodiments, deep neural networks are used as artificial intelligence algorithms, although other types of artificial intelligence algorithms may also be used.

The set/library of data extraction algorithms may be modified, in particular extended, if expedient. That is, if, for example, a new application algorithm is added to a corresponding set/library of application algorithms, which requires a certain additional information as input, corresponding data extraction algorithms may be added. It is, in principle, also conceivable to apply added data extraction algorithms to all qualified images in structured datasets, which still lack the additional information provided by the added data extraction algorithm, to correspondingly complete the set of additional information.

Suitable data extraction algorithms may be applied to medical images at any point in time during, immediately after or later after the acquisition. The information extraction subsystem may be deployed on a centralized or a decentralized infrastructure, or in a coupled or decoupled configuration with respect to the application subsystem. In particular, in a decentralized version, at least a part of the data extraction algorithms suitable for a certain modality may be provided in a corresponding medical imaging device of the modality itself. In preferred embodiments, data extraction algorithms may be executed by a control device of the medical imaging device with which the medical image was acquired such that the structured dataset may be sent directly from the medical imaging device to the image database. However, at least a part of the information extraction subsystem may also be deployed as a part of existent on-premises information technology (IT) infrastructure within a healthcare institution as well as at least a part of the information extraction subsystem may be operated on an external server and/or a cloud, therefore operating outside the boundaries of certain healthcare institutions.

Regarding the application subsystem, it also comprises a stack of application algorithms running on a respective processing infrastructure, using the image database. In this manner, a multitude of population health functions and/or other healthcare analytics applications useful for the care of patients populations or other clinical tasks may be enabled. Generally, in particular, the application subsystem may also comprise at least one processor and at least one storage means.

In concrete embodiments, at least one of the application algorithms provides search and retrieval of structured datasets similar to a current case and/or generates a list of structured datasets for at least one given criterion, in particular relating to a clinical study.

A first group of applications may include search and case retrieval. Similar cases to a current case are searched among the structured datasets, making use of the extracted higher order structured data to enable the efficient search of the relevant/desired sub group of entries within the image database. Lists of patients for whom structured datasets exist in the image database and/or, preferably, representative images are composed that satisfy pre-defined criteria such as having certain findings, for example, a liver tumour, matching demographics (for example age, gender) and/or matching physiological measurements, for example the size of certain organs.

Closely related to applications like search and case retrieval are applications concerning the generation of registries, that is, lists of images and maybe patients fitting predefined registries of interest, for example relating to diabetes, pulmonary hypertension, organic obstructive lung disease and the like. Based on the additional data in the structured datasets, such images and/or patients are identified and flagged as part of the fitting registry.

Preferably, further, at least one application algorithm may comprise a statistical evaluation over multiple structured datasets relevant to a current case and/or performs a comparison of a current case information with at least one additional information of at least one structured dataset or a statistical information derived therefrom. In such a third group of applications, which may also be termed "analytics, distribution and clustering", statistics are computed and data are acquired to enable the analysis of an individual patient or a group of patients in the context of a relevant population, in particular a subgroup of the image database entries. For example, the size of the myocardium of a certain patient can be plotted against the myocardial size of matching patients (age group, body mass index, etc.) including percentiles, cut-offs, and other relevant statistics.

In an especially preferred embodiment, multiple images (and thus structured data sets) related to the same patient, in particular acquired at different points in time, are associated with each other and at least one application algorithm evaluates at least one of the at least one additional information over time. Since the provided image database of structured datasets is image-centric, i.e. has structured datasets relating to specific images of the patient, the stored elements of the image database do not comprise entries focussing on the evolution of one patient. Thus, for example using certain patient information that may already be present as DICOM metadata, structured datasets of the same patient may be associated with each other such that the temporal course of additional information may be derived from the database and used by at least a part of the application algorithms.

In particular, as a fourth group of applications, risk profiles and risk scores may be calculated. Such application algorithms use a combination of extracted higher order information, that is, additional information from the structured datasets, to calculate risk profiles for specific conditions, for example ischemic heart disease. The existing image database is used to develop and test hypothesis of potential risk scores. For example, for each new incoming patient, the risk score for particular diseases may be computed that may be used to optimize his individual care plan.

As a fifth group of applications, predictive applications able to predict diagnosis, clinical path and/or treatment outcome, may be implemented. Such application algorithms involve prediction and may infer clinical relevant hypothesis using the knowledge within the image database together with data-driven algorithms. For example, the length of hospitalization (or more comprehensive clinical path information) can be predicted by comparing finding-related information (and other extracted additional information) with that of similar patients/cases existent within the image database. As an example, the presence and characteristics of air leaks after lung wedge resection can predict the length of hospitalization. The additional information, representing higher order data, acquired over multiple points in time for a certain patient, can be used to predict diagnosis or to rank the most probable diagnostic hypothesis.

The computer program according to the invention may comprise multiple program means, in particular at least partly representing data extraction algorithms and/or application algorithms. The computer program and/or its parts may be loaded into at least one memory of a computing device, in particular the computing device of an analytics system according to the invention. The computer program comprises program means adapted to carry out the steps of a method according to the invention if the computer program is executed in the at least one computing device of the analytics system.

The computer program may be stored on an electronically readable storage medium according to the invention, which thus comprises electronically readable control information stored thereon, which comprises at least one computer program according to the invention and is adapted to perform a method according to the invention if the storage medium is used in at least one computing device of an analytics system according to the invention.

Further details and advantages of the current invention can be taken from the following description of preferred embodiments, taken in conjunction with the drawings, in which
- Fig. 1: shows an embodiment of an analytics system according to the invention, and
- Fig. 2: shows a decentralized implementation of the information extraction subsystem.

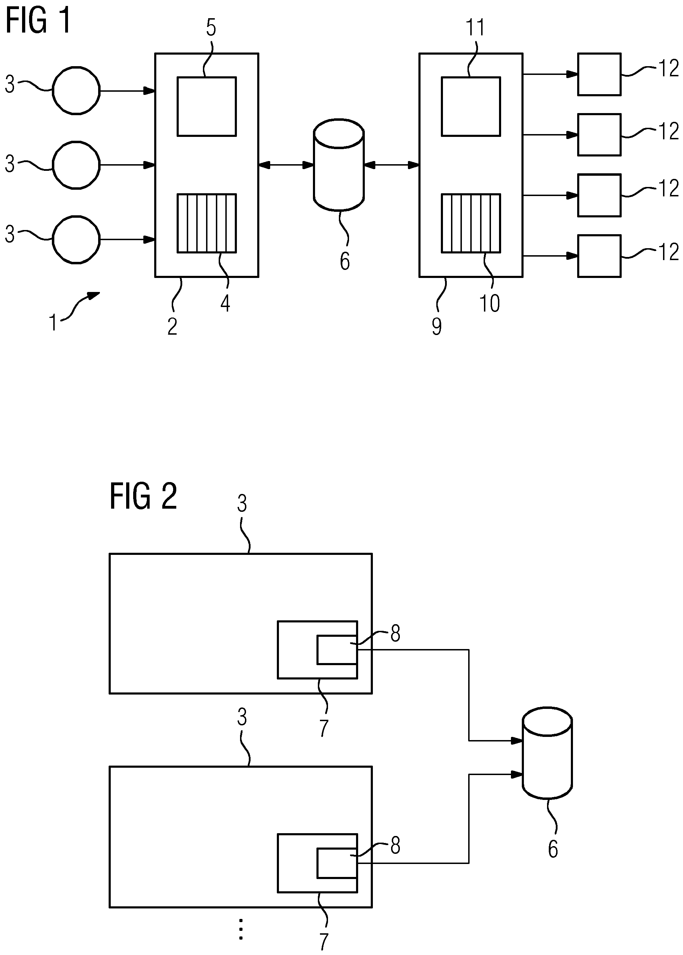

Fig. 1 shows a principle drawing of an analytics system 1 according to the invention. As can be seen, an information extraction subsystem 2 receives medical images from multiple medical imaging devices 3, for example all medical imaging devices of a medical institution or multiple medical institutions. The information extraction subsystem 2 comprises a library 4 having a plurality of data extraction algorithms, at least a part of them being or using artificial intelligence algorithms. All data extraction algorithms of the library 4 may be implemented by corresponding program means. The information extraction subsystem 2 further comprises at least one computing device 5 each having at least one processor and at least one storage means.

The medical imaging device 3 may comprise different imaging modalities, for example a computer tomography device, a magnetic resonance device, an ultrasound imaging device, an x-ray device and/or nuclear imaging device.

Once an examination is completed and a medical image of a patient is received by the information extraction subsystem 2, data extraction algorithms from the library 4 suitable for evaluating the medical image are selected and applied to extract additional information from the medical image. In this embodiment, the medical images are provided in the DICOM format, hence comprising metadata, which is analysed to select suitable data extraction algorithms. The data extraction algorithms, in this embodiment, may, for example, determine additional information comprising geometrical information and/or functional information and/or abnormality information from the medical image. The at least one computing device then combines the medical image, comprising at least part of the DICOM metadata, in particular patient information like age and gender, with the additional information extracted by the data extraction algorithms to form a structured dataset which is then stored in an image database 6 which may, preferably, be hosted in a cloud or at least one server.

Fig. 2 illustrates a preferred decentralized implementation of the information extraction subsystem 2, wherein each medical imaging device 3 comprises a control device 7. Each control device 7 implements a relevant part 8 of the information extraction subsystem 2, in particular using only data extraction algorithms suitable for the modality and type of medical images produced by the medical imaging device 3. In this manner, the structured datasets can be determined in the medical imaging devices 3 themselves and readily be transmitted to the image database 6.

Returning to Fig. 1, the analytics system 1 further comprises an application subsystem 9 in which a library 10 of application algorithms, again at least a part of them being or comprising artificial intelligence algorithms, is provided. The application subsystem 9 again comprises at least one computing device 11, each having at least one processor and a storage means. Each application algorithm in the library 10 relates to a certain health analytics application, in particular population health application. The application algorithms not only analyse and evaluate image data of structured datasets, but also, or, in some cases, exclusively, the additional information of the structured datasets representing higher order data in the image database 6. For example, a user requesting execution of a certain application from a terminal 12 connected to the application subsystem can trigger selection of at least one corresponding application algorithm and its execution in the at least one computing device 11, such that evaluation information is generated and output to the user at the corresponding terminal 12, which also serves as an output unit or device of the analytics system 1.

Applications represented by application algorithms in the analytics system 1 comprise search and retrieval, list generation, in particular registry generation, statistical evaluation and/or comparisons and also applications analysing the progression over time. To this end, structured datasets relating to the same patient, but different examinations at different time points, are associated with each other in the image database 6, such that additional information may be provided for multiple points in time and thus evaluated by at least one application algorithm.

Applications and algorithms using a time series of additional information for the same patient may, for example, comprise the generation of risk profiles and risk scores as well as the prediction of diagnosis, clinical path and/or treatment outcome.

Some application algorithms may, of course, be the basis for application algorithms of a higher order. For example, applications involving statistics may use lists generated by search and case retrieval and/or registry generation application algorithms. For example, in a first step, using a first application algorithm, all structured datasets relating to patients of the same age and sex as the patient of a current case may be found and selected, while in a second step, for example, the size of an organ may be compared to those of these matching patients and/or statistical values determined therefore.

Although the present invention has been described in detail with reference to the preferred embodiment, the present invention is not limited by the disclosed examples from which the skilled person is able to derive other variations without departing from the scope of the invention.

## Claims

1. Method for evaluating images of different patients acquired with multiple medical imaging devices (3), comprising the steps of
- after acquisition of each image, determining at least one additional information from the image using data extraction algorithms, the data extraction algorithms comprising at least one artificial intelligence algorithm, and storing a structured dataset comprising the image and the additional information in an image database (6),
- executing application algorithms using artificial intelligence and evaluating at least the additional information of multiple datasets stored in the image database (6) to determine an evaluation information, and
- outputting the evaluation information to a user.

2. Method according to claim 1, **characterized in that** the additional information comprises geometrical information regarding at least one anatomical structure depicted in the image and/or functional information regarding at least one anatomical structure depicted in the image, in particular derived by a physiological measurement algorithm, and/or at least one abnormality information regarding at least one anatomical structure depicted in the image, in particular comprising at least one diagnostic hypothesis and/or likelihood information,

3. Method according to claim 1 or 2, **characterized in that** at least one patient information describing at least one characteristic of the patient, in particular comprising the age and/or the gender, is additionally stored in the structured dataset, and/or that the image is provided and stored in a DICOM format having metadata, the metadata being used by at least a part of the data extraction algorithms.

4. Method according to one of the preceding claims, **characterized in that** the application algorithms perform at least one population health function.

5. Method according to one of the preceding claims, **characterized in that** at least one of the application algorithms provides search and retrieval of structured datasets similar to a current case and/or generates a list of structured datasets for at least one given criterion, in particular relating to a clinical study.

6. Method according to one of the preceding claims, **characterized in that** at least one application algorithm comprises a statistical evaluation over multiple structured datasets relevant to a current case and/or performs a comparison of a current case information with at least one additional information of at least one structured dataset or a statistical information derived therefrom.

7. Method according to one of the preceding claims, **characterized in that** multiple images related to the same patient, in particular acquired at different points in time, are associated with each other and at least one application algorithm evaluates at least one of the at least one additional information over time.

8. Analytics system (1) for evaluating images of different patients acquired with multiple medical imaging devices (3), comprising
- an information extraction subsystem (2) for receiving the images from the respective medical imaging devices (3), for determining at least one additional information from the image using data extraction algorithms, the data extraction algorithms comprising at least one artificial intelligence algorithm, and for forming a structured dataset comprising the image and the additional information,
- an image database (6) for storing the structured datasets,
- an application subsystem (9) for executing application algorithms using artificial intelligence and for evaluating at least the additional information of multiple datasets stored in the image database (6) to determine an evaluation information, and
- an output unit (12) for outputting the evaluation information to a user.

9. Computer program, which performs the steps of a method according to any of the claims 1 to 7 when the computer program is executed on an analytics system (1).

10. Electronically readable storage medium having a computer program according to claim 9 stored thereon.
